(19) Europäisches Patentamt
European Patent Office
Office européen des brevets

(11) Publication number: **0 024 372 A1**

(12) # EUROPEAN PATENT APPLICATION

(21) Application number: 80302658.2

(22) Date of filing: 04.08.80

(51) Int. Cl.[3]: **C 07 D 498/04**, A 61 K 31/42

(30) Priority: 03.08.79 GB 7927239

(43) Date of publication of application: 04.03.81 Bulletin 81/9

(84) Designated Contracting States: AT BE CH DE FR GB IT LI LU NL SE

(71) Applicant: **BEECHAM GROUP LIMITED, Beecham House Great West Road, Brentford, Middlesex (GB)**

(72) Inventor: **Denerley, Paul Millington, 45 Avondale Close, Horley Surrey (GB)**
Inventor: **Eglington, Alfred John, 66 Nutwood Avenue Brockham, Betchworth Surrey (GB)**
Inventor: **Harbridge, John Barry, 109 St Andrews Road, Coulsdon Surrey (GB)**

(74) Representative: **Hesketh, Alan, Dr. et al, European Patent Attorney Beecham Pharmaceuticals Great Burgh Yew Tree Bottom Road, Epsom, Surrey, KT18 5XQ (GB)**

(54) **Derivatives of clavulanic acid, their preparation, pharmaceutical compositions containing them and their preparation, intermediates and their preparation.**

(57) The compounds of the formula (II):

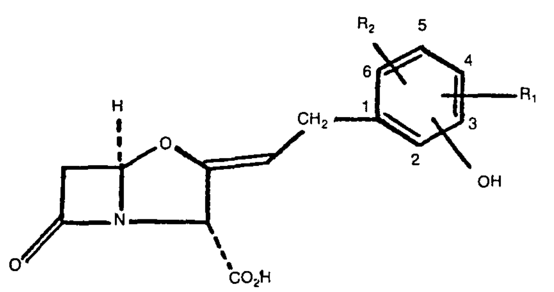

(II)

and salts and esters thereof wherein the hydroxyl group occupies position 2 or 4 $R_1$ and $R_2$ may be the same or different and represent hydrogen, halogen, hydroxy, lower alkyl, cyclopentyl, cyclohexyl, lower alkoxy or $R_1$ and $R_2$ when an adjacent carbon atoms may together represent a 1,4-buta-1,3-dienylene group or a polymethylene group containing from 3 to 5 carbon atoms; have been found to be β-lactam antibiotics and synergists with penicillins and cephalosporins. Their preparation and use is described. The synthesis of intermediates useful in their preparation is also described.

EP 0 024 372 A1

TITLE MODIFIED
see front page

# β-Lactam Antibiotics, their preparation and use

This invention relates to certain 9-phenyldeoxyclavulanic acid derivatives to a method for their preparation and their use as anti-bacterial and β-lactamase inhibitory agents.

British Patent Specification No. 1508977 discloses a compound of formula (I):-

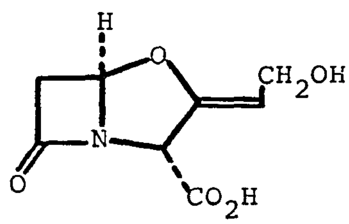

(I)

and salts thereof which are broad spectrum anti-bacterial and β-lactamase inhibitory agents. This compound is now commonly referred to as clavulanic acid.

We have now discovered a series of 9-phenyldeoxyclavulanic acid derivatives which besides being broad spectrum anti-bacterial agents and inhibitors of β-lactamase enzymes have a particularly favourable level of activity against Staphylococci and E. coli. The compounds are therefore of value in the treatment of bacterial infections in man and animals, particularly domestic animals such as cattle, pigs and poultry, especially where the infection is caused by a β-lactamase producing strain of bacteria or where the causal organism is either of the two notably susceptible organisms mentioned above. The compounds are also useful as disinfectants for mineral oils. To whichever of the above medicinal uses the 9-phenyldeoxyclavulanic acid derivatives of this invention

are put, they may be used either alone or with a penicillin or cephalosporin to enhance the activity of these latter antibiotics against resistant microorganisms.

Accordingly the present invention provides a compound of formula (II):-

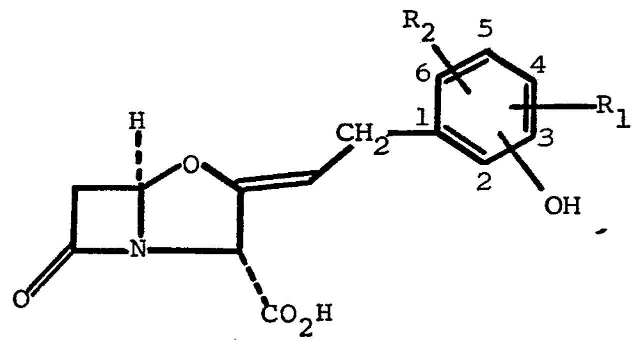

(II)

and salts and esters thereof wherein the hydroxyl group occupies position 2 or 4 $R_1$ and $R_2$ may be the same or different and represent hydrogen, halogen, hydroxy, lower alkyl, cyclopentyl, cyclohexyl, lower alkoxy or $R_1$ and $R_2$ when on adjacent carbon atoms may together represent a 1,4-buta-1,3-dienylene group or a polymethylene group containing from 3 to 5 carbon atoms

When used herein lower alkyl and lower alkoxy mean alkyl and alkoxy groups containing up to four carbon atoms.

Examples of suitable lower alkyl groups falling within the definition of $R_1$ and $R_2$ include methyl and ethyl.

Examples of suitable lower alkoxy groups within the definition of $R_1$ and $R_2$ are methoxy and ethoxy.

Examples of suitable halogen atoms within the definition of $R_1$ and $R_2$ are fluorine, chlorine and bromine, particularly fluorine and chlorine.

One example of a polymethylene group which $R_1$ and $R_2$ may represent is trimethylene.

Most suitably $R_1$ is hydrogen, halogen, hydroxy, lower alkyl or lower alkoxy and $R_2$ is hydrogen. Preferably $R_1$ and $R_2$ are both hydrogen.

The compounds of this invention fall into two sub-groups depending upon the position of the hydroxyl moiety.

The first such sub-group is of formula (IIa):-

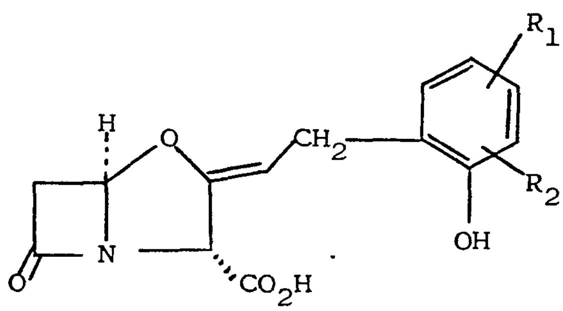

(IIa)

and salts and esters thereof wherein $R_1$ and $R_2$ are as previously defined with reference to formula (II).

Example of this class of compounds are:-

9-(2-hydroxy)phenyldeoxyclavulanic acid,
9-(2-hydroxy)napth-1-yldeoxyclavulanic acid,
9-(6-hydroxy-2-methoxy)phenyl deoxyclavulanic acid,
9-(6-hydroxy-4-methoxy)phenyl deoxyclavulanic acid,
9-(2,4-dihydroxy)phenyldeoxyclavulanic acid,
9-(2,6-dihydroxy)phenyldeoxyclavulanic acid and
9-(3-cyclohexyl-2-hydroxy)phenyldeoxyclavulanic acid.

The second such sub-group is of formula (IIb):-

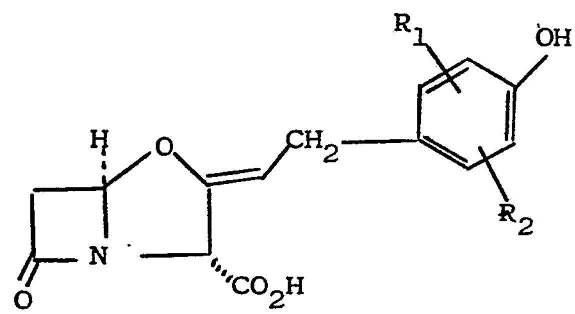

(IIb)

and salts and esters thereof wherein $R_1$ and $R_2$ are as previously defined with reference to formula (II) above.

Examples of this class of compounds are:-

9-(3,5-dimethyl-4-hydroxy)phenyldeoxyclavulanic acid and 9-(3-cyclohexyl-4-hydroxy)phenyldeoxyclavulanic acid.

The compounds of the formula (II) are suitably provided in the form of the free acid. Alternatively the compounds of the formula (II) are provided in the form of a pharmaceutically acceptable salt or in-vivo hydrolysable ester.

Examples of pharmaceutically acceptable salts of the compounds of the formula (II) include the alkali metal salts such as sodium or potassium, alkaline earth metal salts such as calcium or magnesium, and ammonium or substituted ammonium salts for example those with lower alkylamines such as triethylamine, hydroxy-lower alkylamines such as 2-hydroxyethylamine, bis-(2-hydroxyethyl)-amine, tris-(hydroxymethyl)amine or tris-(2-hydroxyethyl)-amine, cycloalkylamines such as bicyclohexylamine, or with procaine, dibenzylamine, N,N-dibenzylethylenediamine, 1-ephenamine, N-ethylpiperidine, N-benzyl-β-phenethylamine, dehydroabietylamine or N,N'-bis-dehydroabietylethylenediamine.

Thus suitable salts of this invention include the sodium, potassium, calcium, magnesium and ammonium salts, of these the sodium, potassium and calcium salts are favoured.

The compounds of the formula (II) may be provided as in-vivo hydrolysable esters. Such esters are those which hydrolyse in the human body to produce the parent acid. Suitable in-vivo hydrolysable esters include those esters known to give in-vivo hydrolysis in penicillins. Thus suitable esters include those of the formula (i):

$$-CO-O-CHR^{a}-O-CO-R^{b} \qquad (i)$$

wherein $R^a$ is a hydrogen atom, or a methyl or phenyl group; $R^b$ is an alkyl group of 1 to 6 carbon atoms,

a phenyl group, an alkyl group of 1 to 3 carbon atoms substituted by a phenyl group, an alkoxy group of 1 to 6 carbon atoms, a phenoxy group, or an alkoxy group of 1 to 3 carbon atoms substituted by a phenyl group; or $R^a$ is attached to $R^b$ to form a 1,2-diphenylene or 4,5-dimethoxy-1,2-diphenylene group.

Favourably $R^a$ is hydrogen.

When $R^a$ is hydrogen suitably $R^b$ is selected from methyl, ethyl, n-propyl, iso-propyl, n-butyl, tert-butyl, phenyl, benzyl, methoxy, ethoxy, n-propyloxy and iso-propyloxy. Preferably $R^b$ is tert-butyl.

Favourably $R^a$ and $R^b$ are joined so that the ester is a phthalidyl or 3,4-dimethoxyphthalidyl ester.

Of these esters those favoured as in-vivo hydrolysable esters are the acetoxymethyl, acetoxyethyl, phthalidyl, ethoxycarbonyloxymethyl, α-ethoxycarbonyloxyethyl and pivaloyloxymethyl esters.

The in-vivo hydrolysable nature of the ester may be confirmed by administration to an animal such as a mouse or rat and determination of the presence of a compound of the formula (II) or a salt thereof in the blood or urine of the animal. Alternatively hydrolysis in human blood or serum may be determined.

It must be realised that salts of the compounds of the formula (II) formed with pharmaceutically unacceptable ions are useful as they may serve as intermediates in the preparation of pharmaceutically acceptable salts by ion-exchange, or they may be useful as intermediates in the preparation of in-vivo hydrolysable esters. An example of such a salt is the lithium salt.

It is appreciated that compounds (II) have at least two acidic groups viz the carboxyl and phenolic hydroxyl groups, but when salts and esters of compounds (II) are referred to salts and esters of the carboxylic acid moiety only are intended.

Compounds (II) may be prepared by arylating a clavulanic acid ester with a phenol in the presence of a Friedel-Crafts catalyst.

Accordingly, the present invention provides a process for preparing a compound of formula (II) above, or a salt or ester thereof, which process comprises arylating a clavulanic acid ester of formula (III) or a derivative thereof that allows arylation to take place:

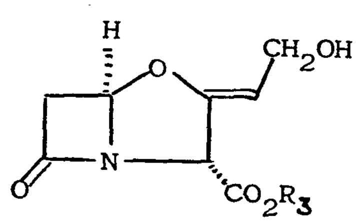

(III)

wherein $R_3$ is an ester forming group, with an appropriate phenol of formula (IV):-

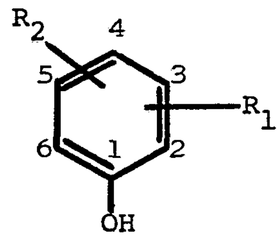

(IV)

wherein $R_1$ and $R_2$ are as defined with reference to formula (II) and position 2 and/or 4 is occupied by a hydrogen atom, in the presence of a Friedel-Crafts catalyst; and thereafter:

a) where desired de-esterifying the ester of formula (II) to form a free acid or its salt;

b) optionally salifying or esterifying the free carboxylic acid if formed in step a);

c) optionally converting the salt if formed in step a) into a free carboxylic acid or an ester.

Any convenient ester of clavulanic acid may be used in the process of this invention. Since it is frequently desirable to form a salt of compounds (II), the ester of clavulanic acid employed is preferably one which is readily converted to the parent acid or its salt by mild methods of hydrolysis or hydrogenolysis. In a further aspect therefore the invention includes a process for preparing a salt or free acid of a compound (II), which process comprises de-esterifying an ester of a compound of formula (II) made by the process of this invention. Particularly suitable esters of clavulanic acid for use in this process include methoxymethyl clavulanate, or a clavulanic acid benzyl ester optionally substituted in the para position by a lower alkyl, lower alkoxy or nitro group or a halogen atom.

A preferred ester for use in this process is benzyl clavulanate.

The phenol (IV) chosen for use in this invention is determined by the particular deoxyclavulanic acid derivative selected for synthesis. Accordingly with reference to formula (IV) examples of suitable lower alkyl groups falling within the definition of $R_1$ and $R_2$

include methyl and ethyl; examples of suitable lower alkoxy groups within the definition of $R_1$ and $R_2$ are methoxy and ethoxy and examples of suitable halogen atoms within the definition of $R_1$ and $R_2$ are fluorine, chlorine and bromine, particularly fluorine and chlorine.

Most suitably $R_1$ is hydrogen, halogen, hydroxy, lower alkyl or lower alkoxy and $R_2$ is hydrogen. Preferably $R_1$ and $R_2$ are both hydrogen.

Examples of suitable Friedel-Crafts catalysts include aluminium trichloride, zinc chloride, zinc bromide, antimony pentachloride, ferric chloride, stannic chloride and boron trifluoride etherate or its chemical equivalent, of which boron trifluoride etherate is preferred.

The reaction is generally carried out in the presence of a solvent, though the identity of the particular solvent selected is not critical to the success of the reaction provided that it is able to form a homogeneous solution of the phenol and the clavulanic acid ester and is substantially inert to the starting materials, product and catalyst. Suitable solvents may be selected by trial and error. We have found generally that the reaction may be conveniently carried out in non-polar organic solvents such as halogenated hydrocarbons e.g. chloroform, carbon tetrachloride, methylene chloride and ethylene chloride.

The temperature at which the reaction is carried out depends upon the particular ester and phenol which have been selected and upon the nature of the catalyst.

Generally, the reaction is carried out at moderate temperatures to low temperatures, i.e. less than 50$^{O}$C and as low as -70$^{O}$C. In order to prevent side reactions, it is preferred to keep the temperature in the range -20$^{O}$ to 0$^{O}$C while the reaction is taking place.

It is of course understood that best results are obtained when reactions of the type described here are carried out under an atmosphere of dry air or a dry inert gas such as argon or nitrogen.

In a further aspect this invention provides a process for the preparation of an ester of a compound of formula (II) as defined above which process comprises rearranging a compound of formula (V):-

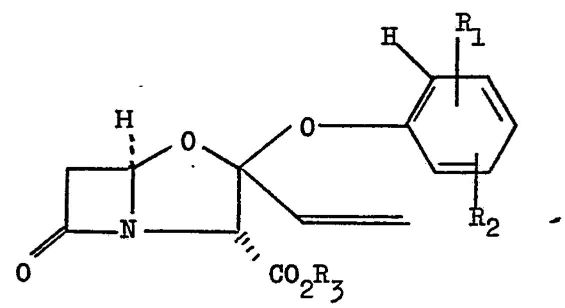

(V)

wherein $R_1$ and $R_2$ are as defined with reference to formula (II) and $R_3$ is an ester forming group.

After the rearrangement step, where desired, the ester of the compound of formula (II) so obtained may be converted into a free acid or a salt.

The rearrangement is generally carried out by heating a solution of compound of formula (V) in an inert high boiling solvent to a moderately high temperature, i.e. generally higher than about 80°C but less than that at which any significant thermal decomposition takes place. Generally the temperature is in the range of 100° - 150°C. Convenient solvents for this process include high boiling aromatic hydrocarbons such as high boiling mono-, di-, or tri - lower alkyl benzenes, examples of which include toluene, xylene and cumene. Xylene is a particularly convenient solvent since it has a boiling point of 140°C and the reaction may conveniently be carried out in this solvent under reflux.

The time for which the reaction is allowed to proceed depends upon the particular starting material selected, but a suitable time period may be easily determined by monitoring the progress of the reaction by for example, high pressure liquid chromatography or more simply by thin layer chromatography on silica using for example ethyl acetate: cyclohexane

as a developing solvent.

$R_3$ may represent any convenient ester group forming group but since it is frequently desirable to form a salt of compounds (II) the group $R_3$ employed is preferably one such that the compound (II) is readily converted into the parent acid or its salt by mild methods of hydrolysis or hydrogenolysis.

Particularly suitable esters of compounds (V) for use in this process include the methoxymethyl ester, or a benzyl ester optionally substituted in the para position by a lower alkyl, lower alkoxy, or nitro group or a halogen atom.

A preferred ester for use in this process is the benzyl ester.

The groups $R_1$ and $R_2$ in compounds (V) are selected according to the requirements of the compound (II) to be produced.

In a further aspect the present invention provides a process for the preparation of compounds of the formula (II) wherein a hydroxyl group is at the 2-position which process comprises reacting a compound of formula (III)

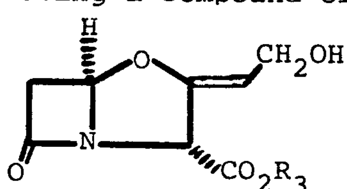

(III)

wherein $R_3$ is the residue of an ester group, with a phenol of formula (IV):-

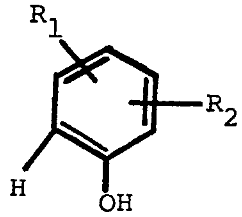

(IV)

wherein $R_1$ and $R_2$ are the same or different and are defined as in relation to formula (II), in the presence of a compound of the formula (VI):-

$$R_4O.CO.N = N.CO.OR_5 \qquad (VI)$$

wherein $R_4$ and $R_5$ are independently lower alkyl, lower alkylaryl or aryl; and a compound of the formula (VII):-

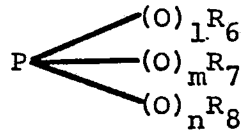

(VII)

wherein l, m and n are independently 0 or 1 and $R_6$, $R_7$ and $R_8$ are each independently a lower alkyl, lower alkylaryl or aryl group; thereafter

a) where desired de-esterifying the ester of formula (II) so formed to form a free acid or its salt;

b) optionally salifying or esterifying the free carboxylic acid formed in step a);

c) optionally converting the salt formed in step a) into a free carboxylic acid or an ester.

The esters of clavulanic acid which may be used in this process are precisely the same as those which may be used in the Friedel-Craft process and the rearrangment process described above.

A preferred ester for use in this process is benzyl clavulanate.

Suitable compounds of the formula (VI) include those wherein the $R_6$, $R_7$ and $R_8$ groups are selected from methyl, ethyl, n-propyl, n-butyl, benzyl, phenyl and methoxyphenyl groups. It is generally convenient that $R_6$, $R_7$ and $R_8$ are the same.

Favoured compounds of the formula (VI) include triarylphosphines and trialkylphosphites.

Particularly suitable compounds of the formula (VI) include triphenylphosphine, trimethylphosphite and triethylphosphite. A further particularly suitable compound of the formula (VI) is tri-p-methoxyphenylphosphine.

Suitable compounds of the formula (V) include those wherein $R_4$ and $R_5$ are independently selected from methyl, ethyl, propyl, butyl, phenyl, benzyl and like groups. It is generally convenient that $R_4$ and $R_5$ are the same.

Particularly suitable compounds of the formula (V) include those wherein $R_4$ and $R_5$ each represent an ethyl or t-butyl group.

In general, roughly equal molar equivalents of the reagents are employed in the process of this invention.

The reaction is performed in an inert organic solvent. The solvent used should be aprotic and unreactive towards the reagents involved. Suitable solvents include tetrahydrofuran, dioxane, ethyl acetate and the like. Other solvents include 1,2-dimethoxyethane and benzene.

A particularly suitable solvent for the process of this invention is tetrahydrofuran.

The reaction is normally carried out at a non-extreme temperature such as $-60^{\circ}C$ to $+100^{\circ}C$, more usually from about $5^{\circ}C$ to about $50^{\circ}C$, for example at approximately ambient temperature (such as about $12^{\circ}C$ to $25^{\circ}C$).

For those reactions using a compound of the formula (VI) wherein l, m, and n are each 0, the reaction is generally complete within a short time, for example normally within 2 hours, usually within 30 minutes and frequently almost immediately. For those reactions using a compound of the formula (VI) wherein l, m and n are each 1, longer reaction times are required, for example up to 24 hours.

The reaction of the compounds of the formula (III), (IV), (VI) and (VII) as hereinbefore defined may also lead to the preparation of an ester of a compound of formula (V), wherein $R_1$ and $R_2$ are defined in relation to a compound of formula (II).

It is to be understood that a number of components are obtained as a result of this process, and that skilled and careful chromatographic techniques are to be employed to isolate the desired products. Compounds of formula (V) may be separated from compounds of formula (II) by gradient elution chromatography (increasing polarity) on silica gel in the first instance, followed by either high pressure liquid chromatography or further column chromatography as appropriate to the chromatographic qualities of the mixture.

The ester of the compound (II) produced in any of these processes may be isolated by any standard method such as fractional crystallisation, counter-current separation or chromatography. We have found that it is most convenient to separate the desired product by column chromatography.

Esters of compounds (II) may be de-esterified by conventional methods such as hydrolysis or hydrogenolysis.

Suitable methods for hydrolysing esters of compounds (II) include mild base hydrolysis in aqueous solution. The reaction may be effected by maintaining the ester at a pH of 7.5 to 9.5 until hydrolysis is complete. Most suitably a readily hydrolysable ester such as the methoxymethyl ester is employed in this process. The pH may be maintained in the desired range in a pH-stat by the addition of a solution of a base such as LiOH, NaOH, KOH, $NaHCO_3$ or the like at a rate that prevents accumulation of excess base which would cause the pH to increase unacceptably or by the use of a suspension of finely divided $Ca(OH)_2$, $Mg(OH)_2$, MgO, $MgCO_3$ or the like. Other bases which may be employed for hydrolysis include $Ba(OH)_2$, $Sr(OH)_2$ and the like.

Suitable methods of hydrogenolysis of esters of compounds (II) include hydrogenation in the presence of a transition metal catalyst. Suitable hydrogenolysable esters are benzyl and substituted benzyl esters discussed above. The pressure of hydrogen used in the reaction may be low, medium or high but in general an approximately atmospheric or slightly super-atmospheric pressure of

hydrogen is preferred. The transition metal catalyst employed is preferably palladium on charcoal. The hydrogenation may be effected in any inert solvent in which the ester is soluble such as tetrahydrofuran or the like. If this hydrogenation is carried out in the presence of a base then a salt of compounds (II) is produced. Suitable bases for inclusion include $NaHCO_3$, $KHCO_3$, $Na_2CO_3$, $K_2CO_3$, $CaCO_3$, $MgCO_3$, $LiHCO_3$, $NH_4OCOCH_3$, $Mg(OCOCH_3)_2$, $Mg(OCOH)_2$ and the like. If no base is present then hydrogenation leads to the preparation of an acid within formula (II) which may then be neutralised if desired to yield a salt. Suitable bases which may be used to neutralise acids within formula (II) include LiOH, NaOH, $NaHCO_3$, KOH, $Ca(OH)_2$, $Ba(OH)_2$, MgO, $Mg(OH)_2$, $NH_4OH$ and $N(C_2H_5)_3$.

The lithium salts within formula (II) tend to be more easily prepared in pure crystalline form than other salts of compounds (II). It is therefore often convenient to first form the lithium salt and then convert this into a further salt by ion-exchange, for example by passing a solution of the lithium salt through a bed of a cation exchange resin in sodium, potassium, calcium, ammonium or like form. Suitable cation exchange resins include Amberlite IR 120 and equivalent resins.

The salts of acid (II) may be converted to esters in conventional manner, for example by reaction with a reactive halide in solution in dimethylformamide or like solvent. Esters may similarly be prepared by the reaction in an inert solvent of an acid (II) with an alcohol in the presence of a condensation promoting agent such as dicyclohexylcarbodiimide.

In general salts of the compounds (II) above may be prepared from the free acid by known methods as disclosed in British Patent Specification No. 1508977 for the preparation of clavulanic acid salts. Similarly, esters of compounds (II) are prepared from the free acid by known methods as disclosed in British Patent Specification No. 1508978 for the preparation of clavulanic acid esters.

In order to use the compounds (II) and their pharmaceutically or veterinary acceptable salts and esters in human or veterinary medicine, these compounds are formulated as pharmaceutical compositions for human or veterinary use in accordance with standard pharmaceutical procedures.

Accordingly the present invention also provides pharmaceutical compositions which comprise a compound of formula (II) or a pharmaceutically acceptable salt or in-vivo hydrolysable ester thereof and a pharmaceutically acceptable carrier.

Suitable forms of the compositions of this invention include tablets, capsules, creams, syrups, suspensions, solutions, reconstitutable powders and sterile forms suitable for injection or infusion. Such compositions may contain conventional pharmaceutically acceptable materials such as diluents, binders, colours, flavours, preservatives, disintegrants and the like in accordance with conventional pharmaceutical practice in the manner well understood by those skilled in the art of formulating antibiotics.

Injectable or infusable compositions of salts of a compound of the formula (II) are particularly suitable as high tissue levels of a compound of the formula (II) can occur after administration by injection or infusion. Thus, one preferred composition aspect of this invention comprises a salt of a compound of the formula (II) in sterile form.

Unit dose compositions comprising a compound of the formula (II) or a salt or ester thereof adapted for oral administration form a further preferred aspect of this invention.

The compound of the formula (II) or its salt or ester may be present in the composition as sole therapeutic agent or it may be present together with other therapeutic agents such as a penicillin or cephalosporin. Suitable penicillins or cephalosporins for inclusion in the compositions of this invention include benzylpenicillin, phenoxymethylpenicillin, carbenicillin, azidocillin,

propicillin, ampicillin, amoxycillin, epicillin, ticarcillin, cyclacillin, cefatriazine, pirbenicillin, α-sulphonyloxybenzylpenicillin, cephaloridine, cephalothin, cefazolin, cephalexin, cephacetrile, cephamandole nafate, cephapirin, cephradine, 4-hydroxycephalexin, cefaparole, cephaloglycine, and other well known penicillins and cephalosporins or pro-drugs therefor such as hetacillin, metampicillin, 4-acetoxyampicillin, the acetoxymethyl, ethoxycarbonyloxymethyl, pivaloyloxymethyl or phthalidyl esters of benzylpenicillin, ampicillin, amoxycillin or cephaloglycine,or the phenyl,tolyl or indanyl α-esters of carbenicillin or ticarcillin or the like. Such compounds are frequently used in the form of a salt or hydrate.

Naturally if the penicillin or cephalosporin present in the composition is not suitable for oral administration then the composition will be adapted for parenteral administration.

When present in a pharmaceutical composition together with a penicillin or cephalosporin, the ratio of a compound of the formula (II) or its salt or ester present to penicillin or cephalosporin present may vary over a wide range of ratios, for example 3:1 to 1:10 and advantageously may be from 2:1 to 1:5 for example, 1:1 to 1:3. The total quantity of antibacterial agents present in any unit dosage form will normally be between 50 and 1500 mg and will usually be between 100 and 1000 mg.

Compositions of this invention may be used for the treatment of infections on <u>inter alia</u>, the respiratory tract, the urinary tract and soft tissues and mastitis in cattle.

Normally between 50 and 3000 mg of the compounds of the invention will be administered each day of treatment but more usually between 100 and 1000 mg of the compounds of the invention will be administered per day, for example as 1-6 doses, more usually 2-4 doses.

The penicillin or cephalosporin in synergistic compositions of this invention will normally be present up

to or at approximately the amount at which it is conventionally used.

Particularly favoured compositions of this invention will contain from 150 to 1000 mg of amoxycillin, ampicillin or a pro-drug therefor and from 50 to 500 mg of a compound of the formula (II) or a salt or *in vivo* hydrolysable ester thereof and more suitably from 200 to 500 mg of amoxycillin, ampicillin or a pro-drug therefor and from 50 to 250 mg of a compound of the formula (II) or a salt or *in vivo* hydrolysable ester thereof.

The materials present in such compositions may be hydrated if desired, for example ampicillin trihydrate or amoxycillin trihydrate may be employed. The weights of the antibiotics in such compositions are expressed on the basis of antibiotic theoretically available from the composition and not on the basis of the weight of pro-drug.

The following Examples illustrate the preparation of compounds in accordance with this invention.

Example 1

The preparation of benzyl 9-(2,4-dihydroxy)phenyldeoxyclavulanate and benzyl 9-(2,6-dihydroxy)phenyldeoxyclavulanate

Benzyl clavulanate (2.9g, 10 mmol) and resorcinol (1.7g, 16 mmol) were stirred in dichloromethane (20 ml) at $0^{o}C$, under an atmosphere of nitrogen. To this was added boron trifluoride etherate (0.3 ml, 2.5 mmol), and the resultant reaction mixture was stirred at ambient temperature for 2 hours. An additional amount of boron trifluoride etherate (0.5 ml, 4 mmol) was added; the reaction stirred for a further 30 minutes, and then worked-up. The dichloromethane solution was decanted off, and the residual gum was triturated with dichloromethane (2 x 50ml). The combined dichloromethane solutions were washed with aqueous sodium bicarbonate (150 ml), washed with water (100 ml), dried ($MgSO_4$), and evaporated in vacuo to afford a gum (1.1g). This gum was chromatographed on a silica gel (70g) column using ethyl acetate and cyclohexane as eluants. Collection of the appropriate fractions as adjudged by t.l.c. afforded benzyl 9-(2,6-dihydroxy)-phenyldeoxyclavulanate (0.07g) as an oil, i.r. (liq film) 3430, 1800, 1742, 1697 and 1605 $cm^{-1}$; n.m.r. ($CDCl_3$) 3.07 (1H, d, J 17 Hz, 6β-H), 3.39 (2H, d, J 8 Hz, 9-$CH_2$), 3.46 (1H, dd, J 17 and 3 Hz, 6α-H), 4.90 (1H, t, J 8 Hz, 8-H), 5.04 (1H, s, 3-H), 5.12 (2H, s, $CH_2$Ph), 5.6 (2H, br m, 2 x OH), 5.71 (1H, d, J 3Hz, 5α-H), 6.3 - 7.3 (8H, m, aromatic protons) p.p.m. Fractions containing a less mobile component, as adjudged by t.l.c. (Rf 1:1 ethyl acetate hexane 0.4) were combined, and evaporated in vacuo to afford benzyl 9-(2,4-dihydroxy)phenyldeoxyclavulanate (0.12g) as an oil. i.r. (liq film) 3420, 1800, 1742, 1700, 1620 and 1605 $cm^{-1}$, n.m.r. ($CDCl_3$) 3.03 (1 H, d, J 17 Hz, 6 β-H), 3.26 (2H, d, J 8 Hz, 9-$CH_2$), 3.44 (1H, dd, J 17 and 3 Hz, 6α-H), 4.78 (1H, t, J 8 Hz, 8-H), 5.05 (1H, s, 3-H), 5.13 (2H, s, $CH_2$Ph), 5.50 (2H, br m, 2 x OH, disappeared on $D_2O$ shake), 5.68 (1H, d, J 3 Hz, 5-H), 6.3 - 7.3 (8H, m, aromatic protons) p.p.m.

Example 2

The preparation of benzyl 9-(6-hydroxy-2-methoxy)phenyldeoxyclavulanate and benzyl 9-(6-hydroxy-4-methoxy)phenyldeoxyclavulanate

Benzyl clavulanate (0.7g) and *m*-methoxyphenol (0.3g) were dissolved in dichloromethane (7 ml) at $0^oC$, under an atmosphere of nitrogen. To this was added boron trifluoride etherate (0.16 ml, 0.5 eq), and the reaction mixture was allowed to rise to ambient temperature over a period of 60 minutes. It was then diluted with dichloromethane (50 ml), washed with aqueous sodium bicarbonate (50 ml), washed with water (50 ml), dried ($MgSO_4$), and evaporated *in vacuo* to afford a gum (0.7g). This gum was subjected to column chromatography on silica gel (60g) using ethyl acetate and cyclohexane as eluants. Appropriate fractions were collected, as adjudged by t.l.c., and evaporated *in vacuo* to afford benzyl 9-(6-hydroxy-2-methoxy)phenyldeoxyclavulanate as an oil (0.02g).

Fractions containing a less mobile component were collected, combined and evaporated *in vacuo* to afford the compound as a slightly impure oil (0.12g). This was rechromatographed on silica gel (12g) using ethyl acetate and cyclohexane as eluants. The appropriate fractions as adjudged by t.l.c. were collected, combined, and evaporated *in vacuo* to afford benzyl 9-(6-hydroxy-4-methoxy)phenyldeoxyclavulanate as an oil (0.02g).

Example 3

The preparation of benzyl 9-(2-hydroxy)phenyldeoxyclavulanate and benzyl 9-(4-hydroxy)phenyldeoxyclavulanate

Benzyl clavulanate (2.9g, 10 mmol) and phenol (0.94g, 10 mmol) were dissolved in dichloromethane (25 ml), at $0^{o}C$, under an atmosphere of nitrogen. To this was added boron trifluoride etherate (0.45 ml, 4 mmol), and the reaction mixture was allowed to warm to ambient temperature over 90 minutes. It was then diluted with dichloromethane (75 ml), washed with aqueous sodium bicarbonate (100 ml), washed with water (100 ml), dried ($MgSO_4$), and evaporated *in vacuo* to afford a gum. This gum was subjected to column chromatography on silica gel (70g) using ethyl acetate and cyclohexane as eluants. The appropriate fractions as adjudged by t.l.c. were collected, combined and evaporated *in vacuo* to afford a gum (1.1g). This gum was rechromatographed on silica gel (80g) using ethyl acetate and cyclohexane as eluants. Initially phenol was eluted, followed by a mobile β-lactam containing impurity. Then appropriate fractions were collected, combined and evaporated *in vacuo* to afford benzyl 9-(2-hydroxy) phenyldeoxyclavulanate as a slightly impure oil (0.083g). The appropriate fractions were then collected containing a less mobile component. These were combined, and evaporated *in vacuo* to afford benzyl 9-(4-hydroxy)phenyldeoxyclavulanate as an oil (0.011g), i.r. (liq film) 3420, 1800, 1750, 1700, 1655, 1605 and 1600 $cm^{-1}$, n.m.r. ($CDCl_3$) 3.00 (1H, d, J 17 Hz, 6β-H), 3.32 (2H, d, J 8 Hz, 9-$CH_2$), 3.45 (1H, dd, J 17 and 3 Hz, 6α-H), 4.75 (1H, t, J 8 Hz, 8-H), 5.04 (1H, s, 3-H), 5.15 (2H, s, $CH_2Ph$), 5.66 (1H, d, J 3Hz, 5α-H), 6.6 - 7.3 (10H, m, aromatic protons and OH) p.p.m.

Example 4

Preparation of benzyl 9-(2-hydroxy)naphth-1-yldeoxyclavulanate

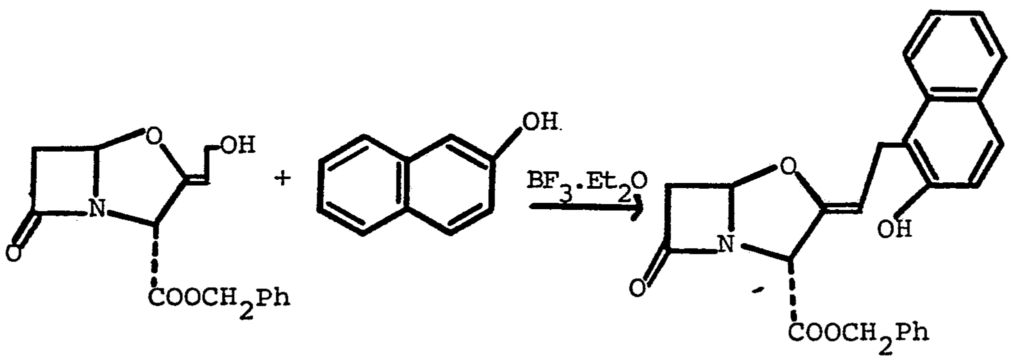


Benzyl clavulanate (2.9g, 10 mmol) and naphth-2-ol (2.9g, 20 mmol) were dissolved in anhydrous dichloromethane (50 ml), at -20$^{o}$C, under an atomsphere of nitrogen. To this stirred solution was added boron trifluoride etherate (0.5 ml, 4 mmol) and the reaction was monitored by t.l.c. The reaction temperature was maintained at 0$^{o}$C. After 3 hours, the reaction mixture was diluted with chloroform (100ml) washed with aqueous sodium bicarbonate (100 ml), dried ($MgSO_4$), and evaporated in vacuo to afford a gum (5.4g). This was subjected to column chromatography on silica gel (65g) using ethylacetate: cyclohexane (1:5). The appropriate fractions were collected, combined, and evaporated in vacuo to afford as a white foam, benzyl 9-(2-hydroxy)naphth-1-yl)deoxyclavulanate (0.76g), i.r. (liq film) 3460, 1800, 1750, 1700, 1630, 1600 $cm^{-1}$, n.m.r. ($CDCl_3$) 3.18 (1H, d J = 17 Hz, 6β-H), 3.45 (1H, dd J = 17 and 3 Hz, 6α-H), 3.75 (2H, d J = 8 Hz, 9-$CH_2$), 4.90 (1H, dt J = 8 and 1 Hz, 8-H), 5.00 (1H, d J = 1 Hz, 3-H), 5.07 (2H, d J ≏ 3 Hz, $CH_2$Ph), 5.65 (1H, d J ≏ 3 Hz, 5-H), 5.66 (1H, br s, OH) disappears on $D_2O$ shake, 7.0 - 7.9 (11H, m, aromatic protons) p.p.m.

Example 5

Preparation of lithium 9-(2-hydroxy)naphth-1-yldeoxy-clavulanate

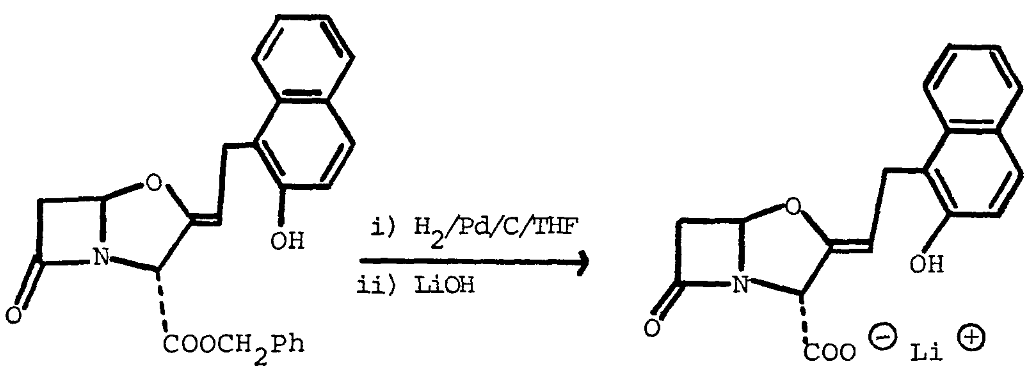


Benzyl 9-(2-hydroxy)naphth-1-yldeoxyclavulanate (0.65g) was dissolved in tetrahydrofuran (20 ml) containing water (10 drops), and was hydrogenated over 10% Palladium on charcoal (0.25g) for 2 hours. The catalyst was filtered off and washed with tetrahydrofuran and water. The combined filtrate and washings were taken to pH 7.0 with aqueous lithium hydroxide. The solvents were then evaporated *in vacuo* to afford a foam (0.6g), that was triturated with ether:acetone (3:1) to afford lithium 9-(2-hydroxy) naphth-1-yldeoxyclavulanate (0.3g) as a solid, i.r. (KBr) 3380, 1805, 1780, 1700, 1600 $cm^{-1}$; n.m.r. ($D_2O$) 2.97 (1H, d $\underline{J}$ = 17 Hz, 6β-$\underline{H}$), 3.4 - 3.8 (3H, m, 6α-$\underline{H}$ and 9-$CH_2$), 8-$\underline{H}$ (obscured by HOD), 5.00 (1H, s, 3-$\underline{H}$), 5.71 (1H, d, $\underline{J}$ ≏ 3 Hz, 5-$\underline{H}$), 7.0 - 7.9 (6H, m, aromatic protons) p.p.m.

Example 6

Benzyl 9-(2-hydroxyphenyl)deoxyclavulanate

Benzyl clavulanate (12.94 g), triphenylphosphine (11.75 g) and phenol (4.2 g) in tetrahydrofuran (150 ml) were cooled in an ice bath and diethyl azodicarboxylate (7.8 g) in tetrahydrofuran (30 ml) was added dropwise to the stirred mixture. The mixture was stirred for a further 30 mins. and the tetrahydrofuran was then evaporated in vacuo. The residue was dissolved in ethyl acetate (ca. 100 ml), silica gel (ca. 15 g) was added to give a slurry and the ethyl acetate was then removed in vacuo to give the reaction product adsorbed onto silica gel. This was slurried in 10% ethyl acetate in petroleum ether (b.p. $60^o - 80^o$) and loaded onto the top of a silica gel column (120 g). The column was eluted with ethyl acetate/petroleum ether (b.p. $60^o - 80^o$) mixtures; 1:9 (250 ml), 15:85 (250 ml), 20:80 (1 litre) and then 30:70. Phenol was eluted first, followed by some mobile β-lactam derivatives, followed by a more polar material (384 mg). This was rechromatographed on silica gel (30 g) eluting with 20% ethyl acetate in cyclohexane (200 ml), followed by 30% EtOAc in cyclohexane. Relevant fractions were combined and evaporated in vacuo to give benzyl 9-(2-hydroxyphenyl) deoxyclavulanate (230 mg). $\nu_{max}(CH_2Cl_2)$ 3570, 1803, 1750, 1701 $cm^{-1}$. $\delta$ $(CDCl_3)$ 3.07 (1H, dd, J 16.5 and 0.8 Hz, 6β-CH), 3.51 (1H, dd, J 16.5 and 3 Hz, 6α-CH), 3.38 (2H, d, J 7.5 Hz, 9-$CH_2$), 4.83 (1H, dt, J 1 and 8 Hz, 8-CH), 5.07 (1H, d, J ca. 1Hz, 3-CH), 5.16 (2H, s, $OCH_2$ Ar), 5.27 (1H, s, exch $D_2O$, OH), 5.73 (1H, dd, J ca.0.8 and 3 Hz, 5-CH), 6.8-7.4 (8H, m, Ar-H) p.p.m.

## Example 7

### Lithium 9-(2-hydroxyphenyl)deoxyclavulanate

Benzyl 9-(2-hydroxyphenyl) deoxyclavulanate (*ca* 180 mg) in tetrahydrofuran (8 ml) containing 10% palladium on barium sulphate catalyst (70 mg) was hydrogenated for 30 minutes at atmospheric pressure and room temperature. The catalyst was then filtered off and washed with tetrahydrofuran. The combined filtrate and washings were diluted with two times their volume of water, and the pH adjusted to 6.8 with 0.1 M aqueous lithium hydroxide. The mixture was evaporated *in vacuo* to small volume, extracted with ethyl acetate, and the aqueous layer was evaporated *in vacuo*. Ethanol was added to the residue and evaporated *in vacuo* (2X), and then toluene was added and evaporated *in vacuo* (2X) to give lithium 9-(2-hydroxyphenyl) deoxyclavulanate as a colourless solid (103 mg). $\nu_{max}$(KBr) 1765, 1680 and 1610 $cm^{-1}$. $\delta$ ($D_2O$) 3.00 (1H, dd, *J* 17 Hz and ca. 1 Hz, 6β-CH), 3.35 (2H, d, *J* 7Hz, 9-$CH_2$), 3.51 (1H,dd, *J* 17 and *ca*. 3Hz, 6α-CH), 4.85 (1H, dt, *J* *ca*. 1 and 7 Hz, 8-CH), 4.86 (1H, broadened s, 3-CH), 5.65 (1H, dd, J *ca*.3 and 1 Hz, 5-CH), 6.75-7.40 (4H, m, Ar-H) p.p.m.

Example 8

Benzyl 9-(6-hydroxy-2-methoxy)phenyldeoxyclavulanate and Benzyl 9-(6-hydroxy-4-methoxy)phenyldeoxyclavulanate.

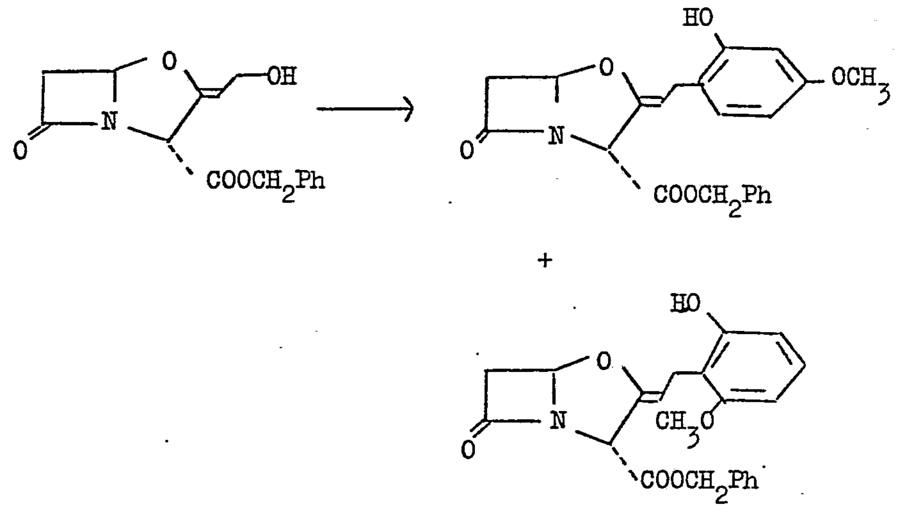

+

Benzyl clavulanate (2.89 g, 10mmol), triphenylphosphine (2.62 g, 10 mmol), and m-methoxyphenol (1.24 g, 10 mmol) were dissolved in tetrahydrofuran (30 ml), and stirred at 0°C. To this reaction mixture was added diethylazodicarboxylate (2.1 ml, 12 mmol). The mixture was stirred for 15 minutes, and evaporated in vacuo to afford a gum which is a mixture of the title compounds. The gum was triturated with ethyl acetate : cyclohexane (20:30 ml), and the filtrate was subjected to column chromatography on silica gel (65 g) using ethyl acetate : cyclohexane.

Fractions which contained mainly benzyl 9-(6-hydroxy-2-methoxy)phenyldeoxyclavulanate as shown by t.l.c., were collected, and combined. The solvent evaporated in vacuo, and the residue was rechromatographed on silica gel (50 g) using ethyl acetate: cyclohexane. Appropriate fractions as shown by t.l.c. were again collected and the solvent was evaporated in vacuo. This afforded benzyl 9-(6-hydroxy-2-methoxy)phenyldeoxyclavulanate as a gum, (0.05 g) i.r. (liq film) 3460, 1800, 1740, 1695, 1605 and 1600 $cm^{-1}$,

n.m.r. ($CDCL_3$): 3.06 (1H, d, *J* 17 Hz 6β-H), 3.38 (2H, d, *J* 7 Hz, 9-$CH_2$), 3.47 (1H, dd, *J* 17 and 2.5 Hz, 6α-H), 3.75 (3H s $OCH_3$), 4.83 (1H, dt, *J* 7 and 1 Hz, 8-H), 5.01 (1H, d, *J* 1 Hz, 3-H), 5.11 (2H, s, $CH_2Ph$), 5.64 (1H, br. s, OH), 5.71 (1H, d, *J* 2.5 Hz, 5-H), 6.4-7.3 (8H, m, aromatic protons). Fractions from the first chromatographic separation which as shown by t.l.c. contained mainly benzyl 9-(6-hydroxy-4-methoxy)phenyldeoxy clavulanate were collected. These fractions were combined, evaporated *in vacuo*, and rechromatographed on silica gel (15 g) using ethyl acetate: cyclohexane to afford as a gum, benzyl 9-(6-hydroxy-4-methoxy)phenyldeoxyclavulanate (0.011 g) i.r. (liq film) 3430, 1800, 1745, 1695, 1610, 1598 $cm^{-1}$ n.m.r. ($CDCL_3$): 3.05 (1H, d, *J* 17 Hz, 6β-H), 3.26 (2H, d, *J* 8 Hz, 9-$CH_2$), 3.47 (1H, dd, *J* 17 and 3 Hz, 6α-H), 3.71 (3H, s, $OCH_3$), 4.78 (1H, t, *J* 8 Hz, 8-H), 5.03 (1H, s, 3-H), 5.12 (2H, s, $CH_2Ph$), 5.70 (1H, d, *J* 3 Hz, 5-H), 6.4-7.3 (8H, m, aromatic protons).

Example 9

Preparation of benzyl 9-(3,5-dimethyl-4-hydroxy)phenyldeoxyclavulanate (VIII)

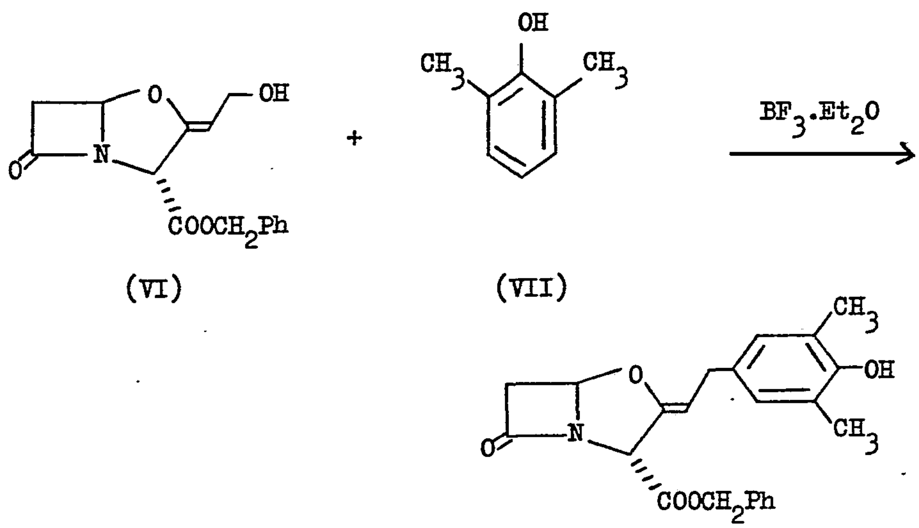


(VI)

(VII)

(VIII)

Benzyl clavulanate (VI) (2.9 g) and 2,6-dimethylphenol (3.6 g) were dissolved in anhydrous dichloromethane (100 ml) at -10°C, under an atmosphere of nitrogen. To this stirred solution was added boron trifluoride etherate (0.6 ml), and the reaction was monitored by t.l.c. After 75 minutes below 0°C, the reaction mixture was diluted with dichloromethane (200 ml), washed with aqueous sodium bicarbonate (150 ml) containing brine (50 ml), dried ($MgSO_4$), and evaporated in vacuo to afford a gum (5.6 g). This gum was subjected to column chromatography on silica gel (70 g) using ethyl acetate : cyclohexane (1:3) as eluent. The appropriate fractions were collected, combined, and evaporated in vacuo to afford benzyl 9-(3,5-dimethyl-4-hydroxy)phenyldeoxyclavulanate (VIII) together with a β-lactam containing impurity, and 2,6-dimethylphenol (VII). This mixture was resubjected to column chromatography on silica gel (70 g) using ethyl acetate : cyclohexane (1:6)→(1:5)→(1:4) as eluent. The appropriate fractions were collected, combined, and

evaporated in vacuo to afford as a gum, benzyl 9-(3,4-dimethyl-4-hydroxy)phenyldeoxyclavulanate (VIII) i.r. (liq film) 1800, 1749, 1701 $cm^{-1}$, n.m.r. ($CDCl_3$) 2.15 (6H, s, $C\underline{H}_3$ x 2), 2.97 (1H, d, J = 17 Hz, 6β-H), 3.26 (2H, d, J = 8 Hz, 9-$C\underline{H}_2$), 3.39 (1H, dd, J = 17 and 3 Hz, 6α-H), 4.75 (1H, dt, J = 8 and 1 Hz, 8-H), 4.83 (1H, br s, OH), 5.05 (1H, d, J = 1 Hz, 3-H), 5.14 (2H, s, $C\underline{H}_2Ph$), 5.65 (1H, d, J = 3 Hz, 5-H), 6.70 (2H, s, 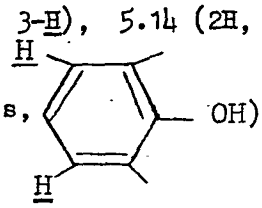), 7.27 (5H, s, $CH_2\underline{Ph}$) p.p.m., together with a β-lactam containing impurity (4:1 mixture).

Example 10

Preparation of lithium 9-(3,5-dimethyl-4-hydroxyphenyldeoxyclavulanate (IX)

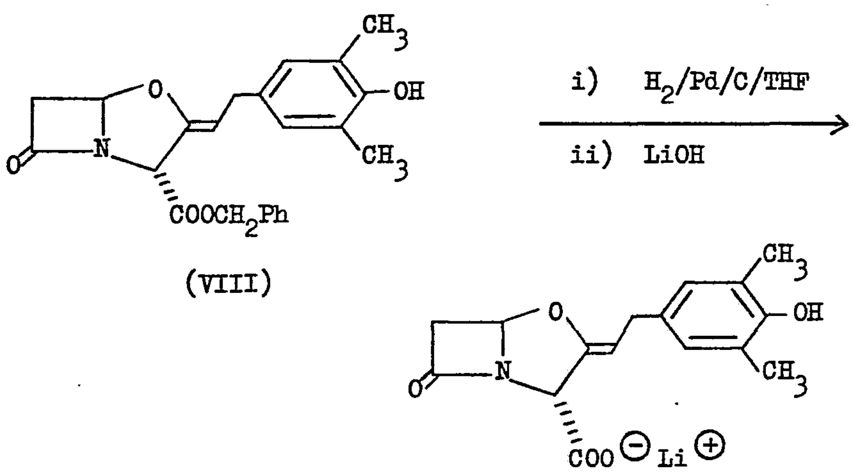


(VIII)

(IX)

The (4:1) mixture of benzyl 9-(3,5-dimethyl-4-hydroxy)phenyldeoxyclavulanate (VIII) and β-lactam containing impurity (from the previous example) (0.10 g) was dissolved in tetrahydrofuran (12 ml) containing water (3 drops), and was hydrogenated over 10% Palladium on Charcoal (0.04 g) for 2 hours. The catalyst was filtered off and washed with tetrahydrofuran and water. The combined filtrate and washings were taken to pH 7.5 with aqueous lithium hydroxide. The solvents then were evaporated in vacuo to afford a gum. This was triturated with ether : acetone (1:1) to afford a white solid. This was washed with acetone : ether (1:3), to afford as a white solid, lithium 9-(3,5-dimethyl-4-hydroxy)phenyldeoxyclavulanate (IX) (0.025 g) i.r. (KBr) 3420, 1810, 1775, 1695, 1605 $cm^{-1}$, n.m.r. ($D_2O$) 2.14 (6H, s, $C\underline{H}_3$ x 2), 3.00 (1H, d, $\underline{J}$ = 17 Hz, 6β-$\underline{H}$), 3.25 (2H, d, $\underline{J}$ = 8 Hz, 9-$C\underline{H}_2$), 3.51 (1H, dd, $\underline{J}$ = 17 and 3 Hz, 6α-$\underline{H}$), 8-$\underline{H}$ (obscured by HOD), 4.84 (1H, s, 3-$\underline{H}$), 5.65 (1H, d, $\underline{J}$ = 3 Hz, 5-$\underline{H}$), 6.80 (2H, br s, aromatic protons) p.p.m.

Example 11

Preparation of p-nitrobenzyl 9-(3-cyclohexyl-2-hydroxy)phenyldeoxy clavulanate (XI) and p-nitrobenzyl 9-(3-cyclohexyl-4-hydroxy)phenyl-deoxyclavulanate (XII)

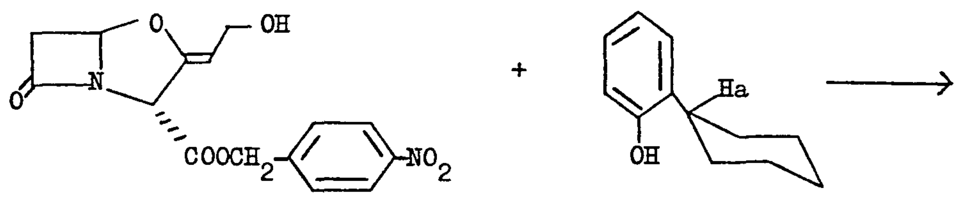

(I) (X)

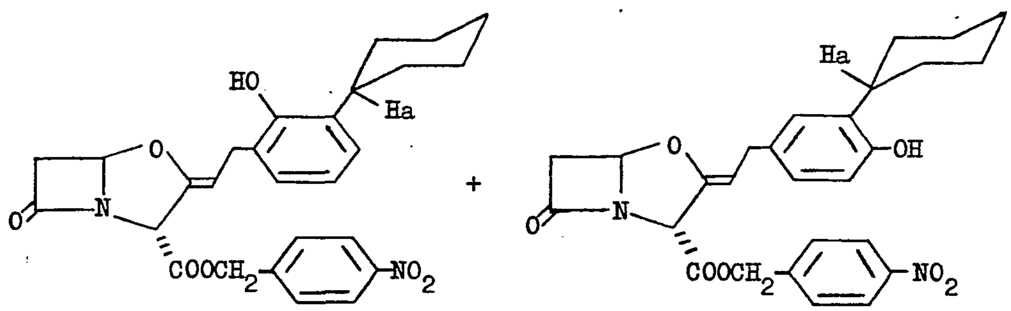

(XI) (XII)

p-Nitrobenzylclavulanate (3.34 g) and 2-cyclohexylphenol (4.8 g) were dissolved in anhydrous dichloromethane (100 ml) at -10°C, under an atmosphere of nitrogen. Boron trifluoride etherate (0.6ml) was added, the temperature was maintained below 0°C. After 90 minutes the reaction mixture was diluted with dichloromethane (200 ml), washed with aqueous sodium bicarbonate (200 ml), dried ($MgSO_4$), and evaporated in vacuo to afford a gum (7.5 g). This gum was subjected to column chromatography on silica gel (65 g) using ethyl acetate : cyclohexane (1:6)→(1:5)→(1:4). The appropriate fractions were collected, combined, and evaporated in vacuo to afford p-nitrobenzyl 9-(3-

cyclohexyl-2-hydroxy)phenyldeoxyclavulanate (XI), i.r. (liq film) 3500, 1800, 1760 $cm^{-1}$, n.m.r. ($CDCl_3$) 1.2 - 2.0 (10H, m, cyclohexyl), 2.7 - 3.0 (1H, m, Ha), 3.12 (1H, d, J = 17 Hz, 6β-H), 3.42 (2H, d, J 8 Hz, 9-$CH_2$), 3.58 (1H, dd, J = 17 and 3 Hz, 6α-H), 4.91 (1H, dt, J = 8 and 1 Hz, 8-H), 5.1 - 5.4 (4H, m, 3-H, $COOCH_2$ and OH), 5.70 (1H, d, J = 3 Hz, 5-H), 6.8 - 8.3 (7H, m, aromatic) p.p.m. contaminated with a β-lactam containing purity (total wt 0.20 g). Subsequent fractions were collected, combined and evaporated *in vacuo* to afford p-nitrobenzyl 9-(3-cyclohexyl-4-hydroxy)phenyl deoxyclavulanate (XII), i.r. (liq film) 3430, 1800, 1755 $cm^{-1}$, n.m.r. ($CDCl_3$) 1.2 - 2.0 (10H, m, cyclohexyl), 2.7 - 3.0 (1H, m, Ha), 3.13 (1H, d, J = 17 Hz, 6β-H), 3.44 (2H, d, J = 8 Hz, 9-$CH_2$), 3.61 (1H, dd, J = 17 and 3 Hz, 6α-H), 4.85 (1H, t, J = 8 Hz, 8-H), 5.1 - 5.4 (4H, m, 3-H, $COOCH_2$ and OH), 5.70 (1H, d, J = 3 Hz, 5-H), 6.8 - 8.3 (7H, m, aromatic) p.p.m. contaminated with the same β-lactam containing impurity as the other isomer (total wt 0.05 g).

Example 12

Preparation of benzyl 9-(2-hydroxy)phenyldeoxyclavulanate

Benzyl 2-phenoxy-2-vinyl clavam-3-carboxylate (0.08 g) was refluxed in xylene (10 ml) at 140°C for 7 hours. The reaction mixture was evaporated in vacuo, and subjected to column chromatography on silica gel (8 g) eluting with 20% ethyl acetate in cyclohexane. The relevant fractions were collected and evaporated in vacuo, to afford benzyl 9-(2-hydroxy)phenyldeoxyclavulanate (0.04 g) as a gum. $\nu_{max}(CH_2Cl_2)$ 3570, 1803, 1750, 1701 $cm^{-1}$. $\delta$ ($CDCl_3$) 3.07 (1H, dd, J 16.5 and 0.8 Hz, 6β-CH), 3.51 (1H, dd, J 16.5 and 3 Hz, 6α-CH), 3.38 (2H, d, J 7.5 Hz, 9-$CH_2$), 4.83 (1H, dt, J 1 and 8 Hz, 8-CH), 5.07 (1H, d, J ca. 1Hz, 3-CH), 5.16 (2H, s, $OCH_2Ar$), 5.27 (1H, s, exch $D_2O$, OH), 5.73 (1H, dd, J ca. 0.8 and 3 Hz, 5-CH), 6.8-7.4 (8H, m, Ar-H) p.p.m.

Example 13

Benzyl 2-Phenoxy-2-vinylclavam-3-carboxylate

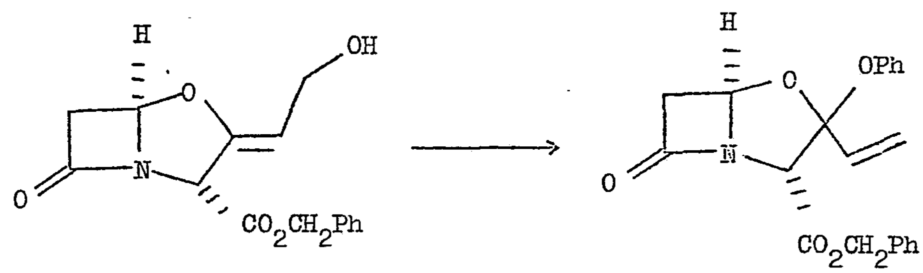

Benzyl clavulanate (12.94g), triphenylphosphine (11.75g) and phenol (4.2g) in tetrahydrofuran (150ml) were cooled in an ice bath and diethyl azodicarboxylate (7.8g) in tetrahydrofuran (30ml) was added dropwise over 10 min. The mixture was stirred for 30 min and then the tetrahydrofuran was removed in vacuo in a rotary evaporator. The residue was taken up in ethyl acetate (ca 100ml), silica gel was added to the mixture and the ethyl acetate was evaporated in vacuo, causing the reaction products to be adsorbed onto the silica gel. This was slurried in petroleum ether (b.p. 60°-80°) containing ethyl acetate (9:1) and the slurry was loaded on to the top of a silica gel column (120g). The column was then eluted with ethyl acetate/petroleum ether (b.p. 60°-80°) mixtures:- 1:9 (250ml), 15:85 (250ml), 2.8 (1l) and then 3:7.

Phenol eluted first, followed by a mixture of phenol, the desired ether and other impurities. This mixture was separated by preparative h.p.l.c. using the two Waters Prep Pak silica gel cartridges, eluting with 10% ethyl acetate in cyclohexane. This separated the phenyl ether and benzyl 2-phenoxy-2-vinylclavam-3-carboxylate from phenol and other impurities to give an approximately 4:1 mixture of benzyl 9-O-phenylclavulanate and benzyl 2-phenoxy-2-vinylclavam-3-carboxylate as an oil (2.8g).

A portion of this mixture (ca.1.3g) was separated by semi-preparative h.p.l.c. using a Whatman Magnum 9 10/25 Partisil column loading 200µl aliquots of an approximately 10% solution of the mixture in ethyl acetate/

cyclohexanc. The column was eluted with 12 to 14% ethyl acetate in cyclohexane at 5ml $min^{-1}$. Combination of the relevant fractions (detected using a Refractive Index detector), and evaporation of the solvent gave benzyl 2-phenoxy-2-vinylclavam-3-carboxylate (193mg), $\nu_{max}$ ($CH_2Cl_2$) 1800, $1765cm^{-1}$, δ ($CDCl_3$) 3.01 (1H, d, J 17Hz, 6β-CH), 3.44 (1H, dd, J 17 and 3 Hz, 6α-CH), 4.64 (1H, s, 3-CH), 5.19 and 5.38 (2H, ABq, J 13.5Hz, $OCH_2$ Ph), 5.31 (1H, dd, J 10 and 2Hz, 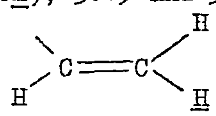 ), 5.33 (1H, dd, J 17 and 2Hz,

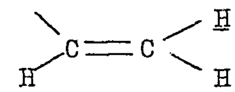 ), 5.72 (1H, d, J 3Hz, 5-CH) 6.01 (1H, dd, J 17 and 10 Hz,

CH=$CH_2$), 6.7-7.50 (10H, m, 10 x Ar-H).

Example 14

p-Methoxybenzyl 2-Phenoxy-2-vinylclavam-3-carboxylate

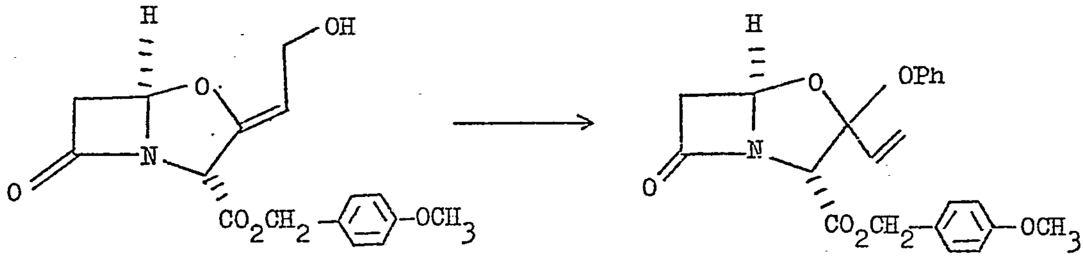

p-Methoxybenzyl clavulanate (650mg), phenol (200mg) and triphenylphosphine (540mg) were stirred together in dry tetrahydrofuran, and cooled in an ice bath. Diethyl azodicarboxylate (0.35ml) was then added. After 2hr. the tetrahydrofuran was removed by means of a rotary evaporator and the residual mixture was chromatographed on silica gel, eluting with ethyl acetate/cyclohexane mixtures (2:8 and then 3:7). This gave a mixture of the 2-phenoxy-2-vinyl clavam and another component (200mg). The mixture was rechromatographed, but separation was not achieved, and the mixture (167mg) was obtained. Ca. two thirds of the mixture was then separated by preparative HPLC. (Whatman Magnum 9 silica gel column, EtOAc/cyclohexane 1:4 elution) to give p-methoxybenzyl 2-phenoxy-2-vinylclavam-3-carboxylate (11mg); $\nu_{max}$ ($CHCl_3$) 1795, 1750cm$^{-1}$; δ ($CDCl_3$) 3.00 (1H, d, J 17Hz, 6β-C$\underline{H}$), 3.43 (1H, dd, J 17 and 3Hz, 6α-C$\underline{H}$), 3.77 (3H, s, OC$\underline{H}_3$), 4.59 (1H, s, 3-C$\underline{H}$), 5.10 and 5.28 (2H, ABq, J 12Hz, OC$\underline{H}_2$Ar), 5.27 (1H, dd, J 10 and ca 1Hz, 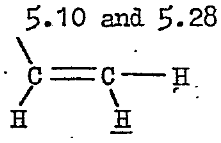 ), 5.51, (1H, dd, J 17 and ca 1Hz, 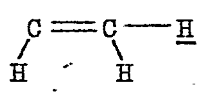 ), 5.68 (1H, d, J 3Hz, 5-C$\underline{H}$), 5.96 (1H, dd, J 17 and 10Hz, C$\underline{H}$=$CH_2$), 6.6-7.4 (9H, m, Ar-H).

Example 15

p-Nitrobenzyl 2-p-formylphenoxy-2-vinylclavam-3-carboxylate

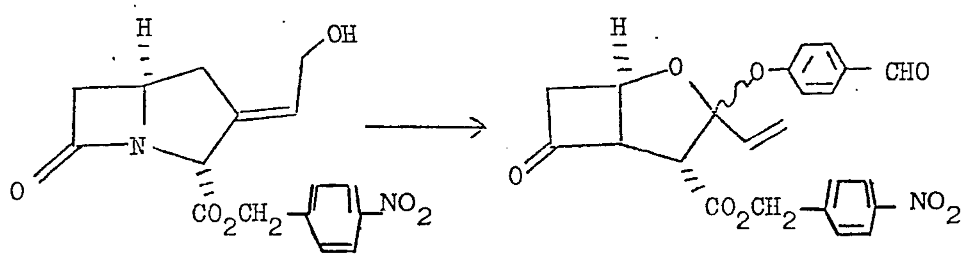

(2 isomers)

p-Nitrobenzylclavulanate (978mg), p-hydroxybenzaldehyde, (400mg), and triphenylphosphine (790mg) in tetrahydrofuran (15ml) were cooled in an ice bath and, whilst stirring, di-tert-butyl azodicarboxylate (690mg) in tetrahydrofuran (5ml) was added. The mixture was allowed to warm to room temperature and then allowed to stir for 45 min. The tetrahydrofuran was then removed by evaporation in vacuo and the residue was chromatographed on silica gel (40g) eluting with ethyl acetate/cyclohexane mixture; 3:7 (200ml), 4:6 (100ml), 1:1. This separated the β-lactam containing material from the di-tert-butyl hydrazodicarboxylate. The β-lactam containing fractions were combined, the solvents removed and the residue was rechromatographed on silica gel (40g) eluting with ethyl acetate/cyclohexane to give p-hydroxybenzaldehyde, followed by 2-p-formylphenoxy-2-vinylclavam-3-carboxylate (isomer A; 70mg) $\nu_{max}$ ($CHCl_3$) 1805, 1760, 1700, 1600$cm^{-1}$, δ ($CDCl_3$) 2.83 (1H, dd, J 17 and ca 1 Hz, 6β-CH), 3.36 (1H, dd, J17 and 3Hz, 6α-CH), 5.07 (1H, s, 3-CH), 5.17 (2H, s, $OCH_2Ar$), 5.31 (1H), dd, J 9Hz and 3Hz, 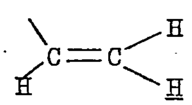 ), 5.59 (1H, dd, J 17 and 3Hz 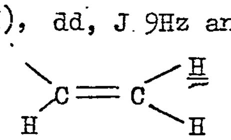 ), 5.84 (1H, m, 5-CH), 5.86 (1H, dd, J 17 and 9 Hz, $CH = CH_2$), 7.10 (2H, d, J 9 Hz, 2 x Ar-H), 7.46 (2H, d, J 9Hz, 2 x Ar-H), 7.10 (2H, d, J 9 Hz, 2 x Ar - H), 8.17 (2H, J 9Hz, 2 x Ar - H), 9.80 (1H, s, ArCHO). Following fractions yielded 2-p-formylphenoxy-2-vinylclavam-3-carboxylate (isomer B, 86mg).

$\nu_{max}$ ($CHCl_3$) 1805, 1767, 1700$cm^{-1}$, $\delta$ ($CDCl_3$) 3.08 (1H, d, J 17Hz, 6β-CH), 3.50 (1H, dd, J 17 and 3 Hz, 6α-CH), 4.60 (1H, s, 3-CH), 5.0-5.8 (5H, m, $OCH_2$ Ar, 5-CH, $CH=CH_2$), 6.03 (1H, dd, J 17 and 10 Hz, $CH=CH_2$), 6.93, 2H, d, J 9Hz, 2 x Ar-H), 7.54 (2H, d, J 9Hz, 2 x Ar-H), 7.65 (2H, d, J 9Hz, 2 x Ar-H), 8.15 (2H, d, J 9Hz, 2 x Ar-H), 9.79 (1H, s, ArCHO).

Demonstration of Effectiveness

In a standard MIC microtitre synergy test with ampicillin the following results were obtained with the compound of Example 7.

| Inhibitor Concentration (μg/ml) | 20 | 5 | 1 |
|---|---|---|---|
| Staphylococcus aureus Russell | - Inhibition - | | |
| Klebsiella aerogenes E70 | 3.1 | 6.25 | 12.5 |
| Proteus mirabilis C889 | 8 | 16 | 16 |
| E.coli JT39 | 2 | 16 | 125 |

WHAT WE CLAIM IS:

1. A compound of the formula (II):

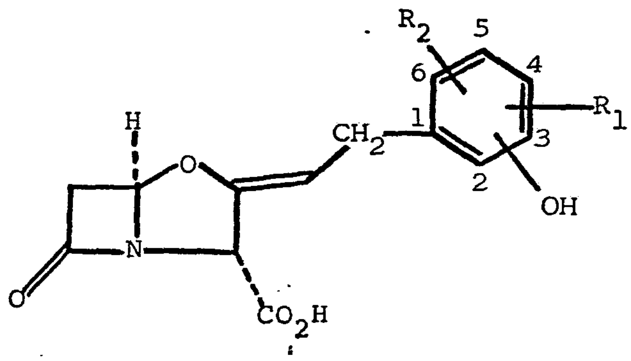

(II)

or a salt or ester thereof wherein the hydroxyl group occupies position 2 or 4, $R_1$ and $R_2$ may be the same or different and represent hydrogen, halogen, hydroxy, lower alkyl, cyclopentyl, cyclohexyl, lower alkoxy or $R_1$ and $R_2$ when on adjacent carbon atoms may together represent a 1,4-buta-1,3-dienylene group or a polymethylene group containing from 3 to 5 carbon atoms.

2. A compound according to claim 1 in the form of a pharmaceutically acceptable salt or in-vivo hydrolysable ester.

3. A compound according to claim 1 selected from:

9-(2-hydroxy)phenyldeoxyclavulanic acid,
9-(2-hydroxy)naphth-lyldeoxyclavulanic acid,
9-(6-hydroxy-2-methoxy)phenyldeoxyclavulanic acid,
9-(6-hydroxy-4-methoxy)phenyldeoxyclavulanic acid,
9-(2,4-dihydroxy)phenyldeoxyclavulanic acid,
9-(2,6-dihydroxy)phenyldeoxyclavulanic acid,

9-(3-cyclohexyl-2-hydroxy)phenyldeoxyclavulanic acid,
9-(4-hydroxy)phenyldeoxyclavulanic acid,
9-(3,5-dimethyl-4-hydroxy)phenyldeoxyclavulanic acid,
9-(3-cyclohexyl-4-hydroxy)phenyldeoxyclavulanic acid,

or pharmaceutically acceptable salts thereof.

4. A compound of the formula (V)

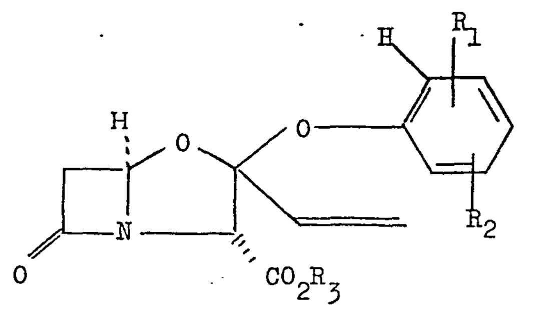

(V)

wherein $R_1$ and $R_2$ are as defined in claim 1 and $R_3$ is an ester forming group.

5. A process for the preparation of a compound of the formula (II) as defined in claim 1, which process comprises arylating a clavulanic acid ester of formula (III) or a derivative thereof that allows arylation to take place:

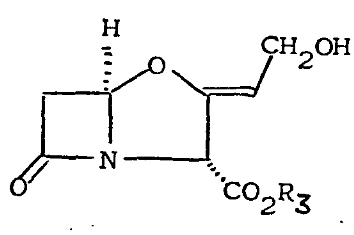

(III)

wherein $R_3$ is an ester forming group, with an appropriate phenol of formula (IV):

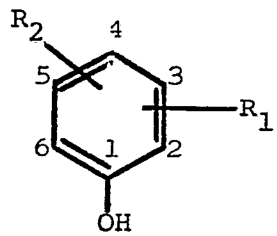

(IV)

wherein $R_1$ and $R_2$ are as defined in claim 1 and position 2 and/or 4 is occupied by a hydrogen atom, in the presence of a Friedel-Crafts catalyst; and thereafter:

a) where desired de-esterifying the ester of formula (II) to form a free acid or its salt;

b) optionally salifying or esterifying the free carboxylic acid if formed in step a);

c) optionally converting the salt if formed in step a) into a free carboxylic acid or an ester.

6. A process for the preparation of an ester of a compound of the formula (II) as defined in claim 1 which process comprises rearranging a compound of the formula (V):

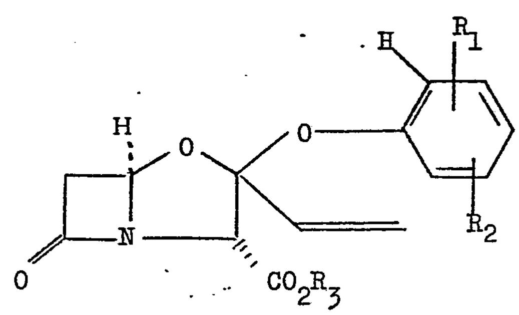

(V)

wherein $R_1$ and $R_2$ are as defined in claim 1 and $R_3$ is an ester forming group.

7. A process for the preparation of a compound of the formula (II) as defined in claim 1 wherein a hydroxyl group is at the 2-position, which process comprises reacting a compound of the formula (III):

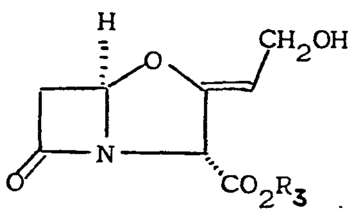

(III)

wherein $R_3$ is the residue of an ester group, with a phenol of formula (IV):

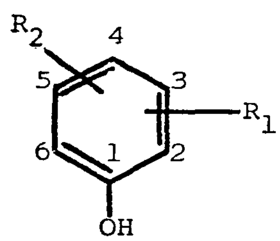

(IV)

wherein $R_1$ and $R_2$ are the same or different and are as defined in claim 1, in the presence of a compound of the formula (VI):

$$R_4O.CO.N{=}N.CO.OR_5 \qquad (VI)$$

wherein $R_4$ and $R_5$ are independently lower alkyl, lower alkylaryl or aryl; and a compound of the formula (VII):-

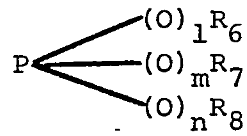

(VII)

wherein l, m and n are independently 0 or 1 and $R_6$, $R_7$ and $R_8$ are each independently a lower alkyl, lower alkylaryl or aryl group; thereafter

a) where desired de-esterifying the ester of formula (II) so formed to form a free acid or its salt;

b) optionally salifying or esterifying the free carboxylic acid formed in step a);

c) optionally converting the salt formed in step a) into a free carboxylic acid or an ester.

8. A process for the preparation of a compound of the formula (V):

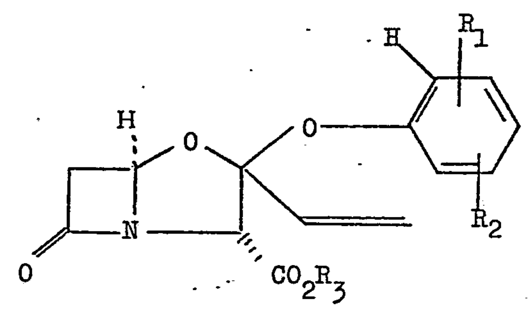

(V)

wherein $R_1$ and $R_2$ are as defined in claim 1 and $R_3$ is an ester forming group, which process comprises the reaction of a compound of the formula (III):

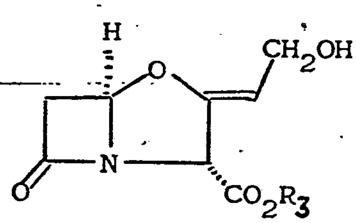

(III)

wherein $R_3$ is the residue of an ester group, with a phenol of formula (IV):

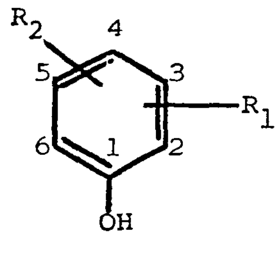

(IV)

wherein $R_1$ and $R_2$ are the same or different and are as defined in claim 1, in the presence of a compound of the formula (VI):

$$R_4O.CO.N{=}N.CO.OR_5 \qquad (VI)$$

wherein $R_4$ and $R_5$ are independently lower alkyl, lower alkylaryl or aryl; and a compound of the formula (VII):-

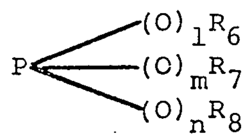

(VII)

wherein l, m and n are independently 0 or 1 and $R_6$, $R_7$ and $R_8$ are each independently a lower alkyl, lower alkylaryl or aryl group; thereafter

a) where desired de-esterifying the ester of formula (II) so formed to form a free acid or its salt;

b) optionally salifying or esterifying the free carboxylic acid formed in step a);

c) optionally converting the salt formed in step a) into a free carboxylic acid or an ester.

9. A pharmaceutical composition which comprises a compound of the formula (II) as defined in claim 1 or a pharmaceutically acceptable salt or in-vivo hydrolysable ester thereof and a pharmaceutically acceptable carrier.

10. A composition according to claim 9 which also comprises a penicillin or cephalosporin.

CLAIMS FOR AUSTRIA

WHAT WE CLAIM IS:

1. A process for the preparation of a compound of the formula (II):

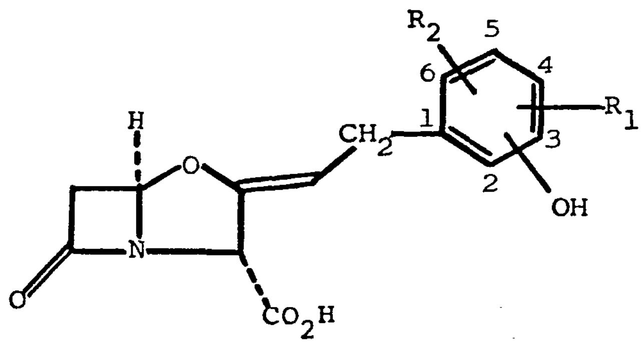

(II)

or a salt or ester thereof wherein the hydroxyl group occupies position 2 or 4, $R_1$ and $R_2$ may be the same or different and represent hydrogen, halogen, hydroxy, lower alkyl, cyclopentyl, cyclohexyl, lower alkoxy or $R_1$ and $R_2$ when on adjacent carbon atoms may together represent a 1,4-buta-1,3-dienylene group or a polymethylene group containing from 3 to 5 carbon atoms; which process comprises arylating a clavulanic acid ester of formula (III) or a derivative thereof that allows arylation to take place:

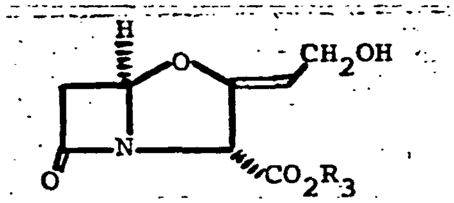

(III)

wherein $R_3$ is an ester forming group, with an appropriate phenol of formula (IV):

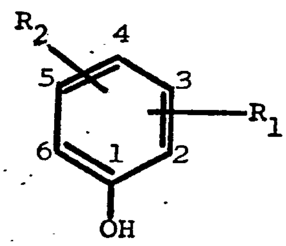

(IV)

wherein position 2 and/or 4 is occupied by a hydrogen atom, in the presence of a Friedel-Crafts catalyst; and thereafter:

a) where desired de-esterifying the ester of formula (II) to form a free acid or its salt;

b) optionally salifying or esterifying the free carboxylic acid if formed in step a);

c) optionally converting the salt if formed in step a) into a free carboxylic acid or an ester.

2. A process according to claim 1 wherein the Friedel-Crafts catalyst is boron trifluoride etherate.

3. A process for the preparation of an ester of a compound of the formula (II) as defined in claim 1 which process comprises rearranging a compound of the formula (V):

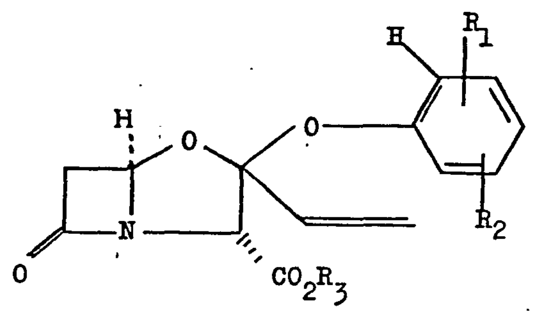

(V)

wherein $R_1$ and $R_2$ are as defined in claim 1 and $R_3$ is an ester forming group.

4. A process according to claim 3 wherein the rearrangement is performed at a temperature of above about 80°C.

5. A process for the preparation of a compound of the formula (II) as defined in claim 1 wherein a hydroxyl group is at the 2-position, which process comprises reacting a compound of the formula (III):

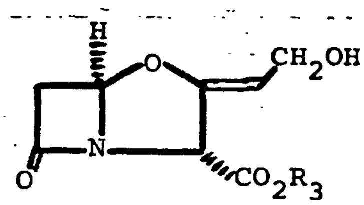

(III)

wherein $R_3$ is the residue of an ester group, with a phenol of formula (IV):

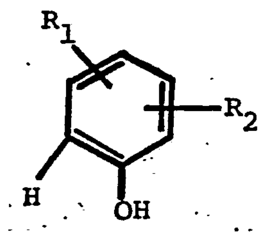

(IV)

wherein $R_1$ and $R_2$ are the same or different and are as defined in claim 1, in the presence of a compound of the formula (VI):

$$R_4O.CO.N{=}N.CO.OR_5 \qquad (VI)$$

wherein $R_4$ and $R_5$ are independently lower alkyl, lower alkylaryl or aryl; and a compound of the formula (VII):-

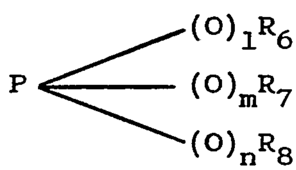

(VII)

wherein l, m and n are independently 0 or 1 and $R_6$, $R_7$ and $R_8$ are each independently a lower alkyl, lower alkylaryl or aryl group; thereafter:

a) where desired de-esterifying the ester of formula (II) so formed to form a free acid or its salt;

b) optionally salifying or esterifying the free carboxylic acid formed in step a);

c) optionally converting the salt formed in step a) into a free carboxylic acid or an ester.

6. A process according to claim 5 wherein the compound of the formula (VI) is diethyl azodicarboxylate.

7. A process according to either claim 5 or claim 6 wherein the compound of the formula (VII) is triphenylphosphine.

8. A process for the preparation of a compound of the formula (V):

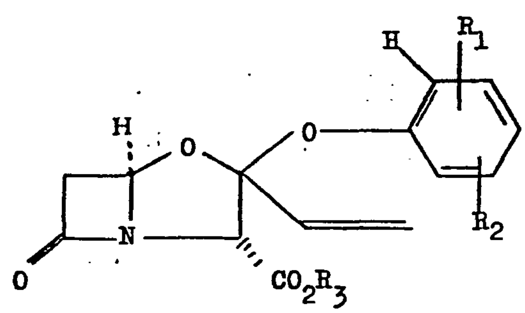

(V)

wherein $R_1$ and $R_2$ are as defined in claim 1 and $R_3$ is an ester forming group, which process comprises the reaction of a compound of the formula (III):

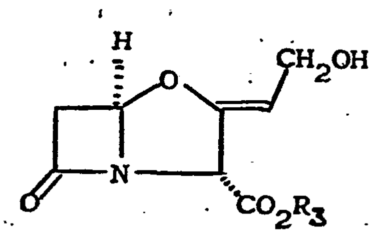

(III)

wherein $R_3$ is the residue of an ester group, with a phenol of formula (IV):

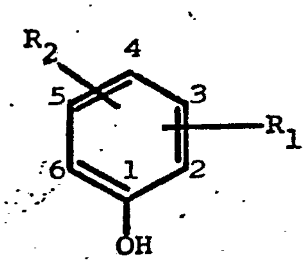

(IV)

wherein $R_1$ and $R_2$ are the same or different and are as defined in claim 1, in the presence of a compound of the formula (VI):

$$R_4O.CO.N{=}N.CO.OR_5 \qquad \text{(VI)}$$

wherein $R_4$ and $R_5$ are independently lower alkyl, lower alkylaryl or aryl; and a compound of the formula (VII);-

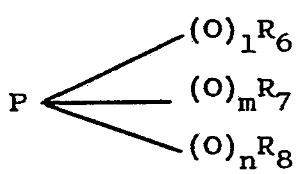

(VII)

wherein l, m and n are independently O or 1 and $R_6$, $R_7$

and $R_8$ are each independently a lower alkyl, lower alkylaryl or aryl group; thereafter:

a) where desired de-esterifying the ester of formula (II) so formed to form a free acid or its salt;

b) optionally salifying or esterifying the free carboxylic acid formed in step a);

c) optionally converting the salt formed in step a) into a free carboxylic acid or an ester.

9. A process for the preparation of a pharmaceutical composition which process comprises bringing into association a compound of the formula (II) as defined in claim 1 or a pharmaceutically acceptable salt or in-vivo hydrolysable ester thereof, with a pharmaceutically acceptable carrier.

10. A process according to claim 9 which also comprises a penicillin or cephalosporin.

European Patent Office

# EUROPEAN SEARCH REPORT

0024372

Application number

EP 80302658.2

| Category | DOCUMENTS CONSIDERED TO BE RELEVANT<br>Citation of document with indication, where appropriate, of relevant passages | Relevant to claim | CLASSIFICATION OF THE APPLICATION (Int. Cl. 3) |
|---|---|---|---|
| | DE - A1 - 2 747 350 (GLAXO)<br>* Pages 1-17 *<br>-- | 1,2,5, 9,10 | C 07 D 498/04<br>A 61 K 31/42 |
| A | DE - A1 - 2 704 104 (BEECHAM)<br>* Pages 1-9, 125, 126, 148, 164, 165 *<br>---- | 1,2,9, 10 | |

TECHNICAL FIELDS SEARCHED (Int.Cl. 3)

C 07 D 498/00
A 61 K 31/00

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons
&: member of the same patent family, corresponding document

X The present search report has been drawn up for all claims

| Place of search | Date of completion of the search | Examiner |
|---|---|---|
| VIENNA | 16-10-1980 | JANISCH |